# EUROPÄISCHE PATENTANMELDUNG

(11) **EP 4 574 803 A1**
(43) Veröffentlichungstag der Anmeldung: **25.06.2025**
(21) Anmeldenummer: 24218100.6
(22) Anmeldetag: 06.12.2024
(51) Int. Cl.: C07C 7/04, C07C 11/02, C07C 45/50, C07C 47/02, C07C 67/38, C07C 69/24

(54) **VERFAHREN ZUR UMSETZUNG VON C11- BIS C20-OLEFINEN MIT VORHERIGER DESTILLATION**

(30) Priorität: 18.12.2023 EP 23217514
(71) Anmelder: Evonik Oxeno GmbH & Co. KG, 45772 Marl (DE)
(72) Erfinder: Brächer, Alexander, 55546 Hackenheim (DE); Franke, Robert, 45772 Marl (DE); Sale, Anna Chiara, 40474 Düsseldorf (DE); Fridag, Dirk, 45721 Haltern am See (DE); Markovic, Ana, 45721 Haltern am See (DE); Knossalla, Johannes, 48351 Everswinkel (DE); Kucmierczyk, Peter, 44628 Herne (DE)
(74) Vertreter: Evonik Patent Association

(57) **Zusammenfassung**

Die vorliegende Erfindung betrifft ein Verfahren zur Umsetzung von C11- bis C20-Olefinen, bei dem der eingesetzte Feedstrom, der zumindest lineare Isomere und verzweigte Isomere der eingesetzten Olefine enthält, vor der Umsetzung destilliert wird und nur die dabei anfallende Schwersiederphase der Umsetzung, einer heterogen katalysierten Hydroformylierung, einer homogen katalysierten Hydroformylierung oder einer homogen katalysierten Alkoxycarbonylierung unterworfen wird.

## Beschreibung

Gegenstand der Erfindung ist ein Verfahren zur Umsetzung von C11- bis C20-Olefinen, bei dem der eingesetzte Feedstrom, der zumindest lineare Isomere und verzweigte Isomere der eingesetzten Olefine enthält, vor der Umsetzung destilliert wird und nur die dabei anfallende Schwersiederphase der Umsetzung, einer heterogen katalysierten Hydroformylierung, einer homogen katalysierten Hydroformylierung oder einer homogen katalysierten Alkoxycarbonylierung unterworfen wird.

Unter dem Begriff "Umsetzung" werden im Rahmen der vorliegenden Erfindung die drei unterschiedlichen Reaktionen heterogen katalysierte Hydroformylierung, homogen katalysierte Hydroformylierung und homogen katalysierte Alkoxycarbonylierung verstanden. Diese drei Reaktionstypen sind für sich bekannte Verfahren der organischen Chemie, werden im Rahmen der vorliegenden Erfindung jedoch ausführlicher beschrieben.

Bei der Hydroformylierung werden Olefine mit Synthesegas, d. h. einer Mischung aus Kohlenmonoxid (CO) und Wasserstoff (H₂), in Gegenwart eines geeigneten homogenen Katalysatorsystems zu den entsprechenden Aldehyden umgesetzt. Bei der Hydroformylierung von C11- bis C20-Olefinen entstehen Aldehyde. Wird beispielsweise Tributen als Olefin eingesetzt, entsteht Isotridecanal. Die Hydroformylierung ist ein großtechnisch genutztes Verfahren, welches in Anlagen betrieben wird, in denen die Aldehyde im Maßstab von mehreren bis hunderten Kilotonnen (kt) pro Jahr produziert werden können. Dabei typischerweise eingesetzte Katalysatorsysteme sind homogen gelöste Katalysatorsysteme und umfassen Übergangsmetallkomplexe aus zumeist Cobalt oder Rhodium als Metall und phosphorhaltigen Liganden. Beispiele dafür sind in der Patentliteratur zahlreich zu finden. Der erhaltene Aldehyd wird in der industriellen Chemie in aller Regel zum Alkohol hydriert. Wird hier das aus dem Tributen erhaltene Isotridecanal eingesetzt, führt die Hydrierung zu Isotridecanol (ITDA).

In den letzten Jahren wurden zudem wieder heterogen katalysierten Hydroformylierungen interessanter. Ein Beispiel für entsprechende Hydroformylierungen sind in der EP 3 632 885 A1 offenbart. Dort werden statt den bekannten homogen gelösten Katalysatoren Katalysatorsysteme eingesetzt, die heterogenisiert auf einem Monolith-Support aus einem porösen keramischen Material vorliegen. Die heterogenisierten Katalysatorsysteme sind insbesondere die aus der homogen katalysierten Hydroformylierung bekannten oder vergleichbare Übergangsmetallkomplexe aus zumeist Cobalt oder Rhodium als Metall und phosphorhaltigen Liganden.

Die Alkoxycarbonylierung ist die Umsetzung eines Olefins mit Kohlenmonoxid und einem Alkohol zu den daraus resultierenden Estern. Dabei werden üblicherweise Metall-Ligand-Komplexe als Katalysatoren eingesetzt, die homogen gelöst im Reaktionsgemisch und damit auch im Produktgemisch vorliegen. Ein entsprechendes Verfahren ist beispielsweise in der EP 3 750 620 A1 offenbart. Die eingesetzten Katalysatorsysteme umfassen insbesondere ein Metall aus der Gruppe 8 bis 10 des Periodensystems der Elemente (PSE), beispielsweise Palladium, oder eine Verbindung davon, einen phosphorhaltigen Liganden und eine Säure als Co-Katalysator.

Um die vorgenannten Umsetzungsverfahren, d. h. die homogen katalysierte Hydroformylierung, die heterogen katalysierte Hydroformylierung oder die homogen katalysierte Alkoxycarbonylierung, wirtschaftlich betreiben zu können kommt es auf verschiedene Faktoren an. Einer dieser Faktoren ist die Reaktionsgeschwindigkeit, die möglichst hoch sein sollte. Schließlich lässt sich im Vergleich zu einer Reaktion mit einer geringeren Reaktionsgeschwindigkeit pro Zeiteinheit mehr Produkt herstellen. Dabei ist aber zu beachten, dass sich bei einer Erhöhung der Reaktionsgeschwindigkeit andere Reaktionsparameter, wie der Reaktionsumsatz und/oder die Selektivität zum gewünschten Produkt sich nicht verschlechtern, damit sich der Vorteil der höheren Reaktionsgeschwindigkeit nicht ins Gegenteil verkehrt.

Die Aufgabe der vorliegenden Erfindung war deshalb die Bereitstellung eines Verfahrens zur Umsetzung von C11- bis C20-Olefine, mit dem eine höhere Reaktionsgeschwindigkeit erzielt werden kann, während sich der Reaktionsumsatz und/oder die Selektivität zum gewünschten Produkt, hier ein Aldehyd bei der Hydroformylierung oder ein Ester bei der Alkoxycarbonylierung, nicht signifikant verschlechtern.

Die Aufgabe konnte durch das Verfahren gemäß den Ansprüchen gelöst werden. Bevorzugte Ausgestaltungen sind in den abhängigen Ansprüchen angegeben. Das erfindungsgemäße Verfahren ist ein Verfahren zur Umsetzung von C11- bis C20-Olefinen, vorzugsweise von C12-, C16- oder C20-Olefinen, besonders bevorzugt von Tributen, welches die folgenden Schritte umfasst:
a) Bereitstellen eines C11- bis C20-Olefin-Feedstroms, vorzugsweise eines C12-, C16- oder C20-Olefin-Feedstroms, besonders bevorzugt eines Tributen-Feedstroms, der zumindest lineare und verzweigte Isomere der eingesetzten Olefine umfasst;
b) Destillation des bereitgestellten Feedstroms in mindestens einer Destillationskolonne unter Erhalt zumindest einer Leichtsiederphase und einer Schwersiederphase, wobei 1 Gew.-% bis weniger als 50 Gew.-% des destillierten Feedstroms als Leichtsiederphase anfällt und wobei der Anteil an linearen Isomeren in der Leichtsiederphase geringer ist als der Anteil der linearen Isomere in der Schwersiederphase;
c) Zuführen der Schwersiederphase aus Schritt b) zu einer Reaktionseinheit, die einen oder mehrere Reaktoren umfasst, und Durchführen einer Umsetzung in der Reaktionseinheit, wobei die Umsetzung entweder eine heterogen katalysierte Hydroformylierung in Anwesenheit von Synthesegas, eine homogen katalysierte Hydroformylierung in Anwesenheit von Synthesegas oder eine homogen katalysierte Alkoxycarbonylierung in Anwesenheit von Kohlenmonoxid und einem C1- bis C6- Alkohol ist.

Der entscheidende Verfahrensschritt ist dabei Schritt b), die Destillation des Feedstroms und die ausschließliche Verwendung der Schwersiederphase für die Umsetzung in Schritt c). Das erfindungsgemäße Verfahren hat den Vorteil, dass die Reaktionsgeschwindigkeit in der Umsetzung in Schritt c) höher ist als bei den bekannten Verfahren, bei denen keine derartige Destillation erfolgt. Grund dafür dürfte sein, dass bei der Destillation eher unreaktivere Isomere und/oder reaktionshemmende Stoffe mit der Leichtsiederphase aus dem Feedstrom abgetrennt und damit nicht der Umsetzung zugeführt werden.

Der erste Schritt a) des erfindungsgemäßen Verfahrens ist die Bereitstellung eines C11- bis C20-Olefin-Feedstroms, vorzugsweise eines C12-, C16- oder C20-Olefin-Feedstroms, besonders bevorzugt eines Tributen-Feedstroms. Die eingesetzten Feedströme sind üblicherweise technisch verfügbare Kohlenwasserstoffströme, die zumindest verschiedene Isomere des jeweiligen Olefins enthalten. Die Feedströme enthalten dabei zumindest lineare Isomere und verzweigte Isomere. Isomere sind bekanntermaßen Olefine mit jeweils gleicher Anzahl an Kohlenstoffatomen, die unterschiedlich aufgebaut sind. Die Menge an den jeweiligen Olefinen in den Kohlenwasserstoffströmen sollte verständlicherweise ausreichend hoch sein, um die Umsetzung in Schritt c) wirtschaftlich betreiben zu können, vorzugsweise sollten die Feedströme mindestens 5 Gew.-% der betreffenden Olefine enthalten, bezogen auf das Gesamtgewicht des Feedstroms.

Olefine mit 10 oder mehr Kohlenstoffatomen können insbesondere durch Oligomerisierungsreaktionen, beispielsweise Dimerisierung, Trimerisierung oder Tetramerisierung, gewonnen werden. Geeignete Kohlenwasserstoffströme sind das bei der Tetramerisierung von Propen oder der Trimerisierung von Butenen anfallende Gemisch isomerer Dodecene (Tetrapropen oder Tributen), das bei der Tetramerisierung von Butenen anfallende isomere Hexadecen (Tetrabuten) sowie durch Cooligomerisierung von Olefinen mit unterschiedlicher Kohlenstoffatomanzahl (bevorzugt 2 bis 4 C-Atome) hergestellte Olefinmischungen, gegebenenfalls nach destillativer Trennung in Fraktionen mit gleicher oder unterschiedlicher C-Zahl. Weiterhin können Olefine oder Olefinmischungen, die durch Fischer-Tropsch-Synthese erzeugt worden sind, eingesetzt werden. Darüber hinaus können Olefine, die durch Olefin-Metathese oder durch andere technische Prozesse hergestellt worden sind, verwendet werden.

Tributen bezeichnet eine Mischung von zumindest verschiedenen linearen und verzweigten Isomeren des Tributens, also C12-Olefinen. Tributen kann insbesondere durch die Oligomerisierung bzw. die Trimerisierung von Butenen erhalten werden, gegebenenfalls nach destillativer Abtrennung aus einem Olig omerisie ru ngsgemisch.

Die in Schritt a) bereitgestellten Feedströme müssen folglich mittels Oligomerisierung oder anderen Verfahren hergestellt werden. Die dabei erhaltenen Reaktionsgemische enthalten oft mehrere unterschiedliche Fraktionen von Olefinen mit unterschiedlicher Kettenlänge. Bei der Oligomerisierung von Butenen entstehen beispielsweise C8-, C12-, C16- und ggf. auch C20-Olefine. Um die erfindungsgemäßen Ströme bereitzustellen, muss vorher also eine Fraktionierung stattfinden, um möglichst reine Fraktionen der Olefine mit jeweils gleicher Kettenlänge zu erhalten, die dann im vorliegenden Verfahren eingesetzt werden. Entsprechende Verfahren sind dem Fachmann bekannt.

Die Feedströme, die in Schritt a) bereitgestellt werden, können vorher einem oder mehreren weiteren Verfahrensschritten unterworfen werden, um bestimmte Komponenten der Feedströme umzusetzen oder aus dem Strom zu entfernen. Ein Beispiel ist die Entfernung von Verunreinigungen, die schädlich für den Katalysator sein können, beispielsweise Sauerstoff-, Stickstoff- oder Schwefel-enthaltende Stoffe oder Verbindungen. Eine Möglichkeit zur Entfernung derartiger Verunreinigungen ist das Überleiten des Feedstroms über ein Adsorberbett, wobei die Verunreinigungen im Adsorber hängen bleiben. Solche Prozesse sind bekannt und zahlreich publiziert worden.

Der in Schritt a) bereitgestellte C11- bis C20-Olefin-Feedstrom, vorzugsweise C12-, C16- oder C20-Olefin-Feedstrom, besonders bevorzugt Tributen-Feedstrom wird im Schritt b) einer Destillation unterworfen, bei der eine Schwersiederphase und eine Leichtsiederphase anfallen. Die Destillation in Schritt b) wird vorzugsweise bei einer Kopftemperatur im Bereich von 40 bis 100 °C, besonders bevorzugt im Bereich von 55 bis 80 °C durchgeführt. Der Druck am Kopf beträgt bei der Destillation in Schritt b) vorzugsweise 50 bis 250 mbar, besonders bevorzugt 80 bis 150 mbar. Die Temperatur im Sumpf beträgt bei der Destillation in Schritt b) vorzugsweise 90 bis 150 °C, besonders bevorzugt 110 bis 140 °C. Der Druck im Sumpf beträgt bei der Destillation in Schritt b) vorzugsweise 70 bis 300 mbar, besonders bevorzugt 90 bis 180 mbar.

Bei der Destillation können unreaktivere Isomere und/oder reaktionshemmende Stoffe zumindest teilweise in die Leichtsiederphase übergehen, wodurch die Umsetzung der Schwersiederphase in Schritt c) mit höherer Rektionsgeschwindigkeit erfolgen kann. Bei der Destillation in Schritt b) fallen 1 Gew.-% bis weniger als 50 Gew.-%, vorzugsweise 5 Gew.-% bis 40 Gew.-%, besonders bevorzugt 10 Gew.-% bis 30 Gew.-% des destillierten Feedstroms als Leichtsiederphase an. Die Abtrennung der Leichtsiederphase in der Destillation kann dabei auf unterschiedliche Art erfolgen, was dem Fachmann grundsätzlich geläufig ist. So kann die Abtrennung der bestimmten Menge der Leichtsiederphase beispielsweise über die Trennschärfe der Destillation, die anhand des Rücklaufverhältnisses und/oder der Temperatur beeinflusst werden kann, erfolgen. Möglich wäre auch eine Abtrennung nach der abgetrennten Masse, also beispielsweise einem Massenmesser oder ähnlichen geeigneten Apparaturen oder Einbauten. Der Anteil oder die Menge an Feedstrom, der oder die als Leichtsiederphase in der Destillation in Schritt b) abgetrennt wird, kann aber auch anhand anderer Parameter eingestellt werden.

Die Folge der Destillation in Schritt b) ist, dass der Anteil an linearen Isomeren der eingesetzten C11- bis C20-Olefine, vorzugsweise der eingesetzten C12-, C16- oder C20-Olefine, besonders bevorzugt des eingesetzten Tributens in der Leichtsiederphase geringer ist als der Anteil der betreffenden linearen Isomeren in der Schwersiederphase. Dies kann beispielsweise mittels NMR oder Gaschromatographie überprüft bzw. ermittelt werden. Die verzweigten Isomere sind üblicherweise leichtsiedender und gehen in größerem Anteil in die Leichtsiederphase über. Da es sich im Rahmen der vorliegenden Erfindung bei den eingesetzten Olefinen um ein Gemisch isomerer Olefine handelt, ist es kaum zu verhindern, dass auch lineare Isomere in die Leichtsiederphase übergehen. Die Destillation sollte jedoch nur in geeigneten Bedingungen durchgeführt werden, um zu ermöglichen, dass der Anteil der linearen Isomere in der Schwersiederphase höher ist.

Die Destillation in Schritt b) wird in mindestens einer Destillationskolonne durchgeführt, kann also sowohl in einer als auch in mehreren Destillationskolonnen durchgeführt werden. Sofern nur eine einzige Destillationskolonne vorhanden ist, wird der für die Umsetzung in Schritt c) benötigte Strom als Sumpfstrom aus der Destillationskolonne abgenommen. Sind mehrere Destillationskolonnen vorhanden, wird nur die Schwersiederphase der ersten Destillationskolonne zur nächstfolgenden Destillationskolonne und die Leichtsiederphase der letzten Destillationskolonne zur Umsetzung in Schritt c) geführt.

Für die erfindungsgemäße Destillation eignen sich grundsätzlich alle bekannten Destillationskolonnen. Diese weisen typischerweise Einbauten zur Verbesserung der Trennschärfe auf. Geeignete Einbauten sind zum Beispiel Böden, unstrukturierte Packungen (Füllkörper) oder strukturierte Packungen. Als Böden werden üblicherweise Glockenböden, Siebböden, Ventilböden mit festen oder beweglichen Ventilen, Tunnelböden oder Schlitzböden eingesetzt. Unstrukturierte Packungen sind im Allgemeinen Füllkörperschüttungen. Als Füllkörper werden üblicherweise Raschigringe, Pallringe, Berl-Sättel, SuperRinge/SuperRinge Plus oder Intalox^{®}-Sättel verwendet. Strukturierte Packungen werden zum Beispiel unter dem Handelsnamen Mellapak^{®} der Firma Sulzer vertrieben. Neben den genannten Einbauten sind dem Fachmann weitere geeignete Einbauten bekannt und können ebenfalls verwendet werden. In einer bevorzugten Ausführungsform umfasst die mindestens eine Destillationskolonne 20 bis 100 Böden, besonders bevorzugt 30 bis 80 Böden.

Die Bedingungen der Destillation sind abhängig von der Feedzusammensetzung und in weiten Bereichen variabel. Für sehr reine Ströme, die nur geringe Anteile an oder keine Verbindungen mit mehr oder weniger Kohlenstoffatomen aufweisen, reicht üblicherweise eine einstufige Destillation aus. Im Sumpf fallen bei der Destillation dann die eher linearen Isomere an, die im Schritt c) umgesetzt werden sollen. Am Kopf der einstufigen Destillation fallen die eher verzweigten Isomere an, die abgetrennt werden. Enthalten die eingesetzten Feedströme signifikante Mengen an Verbindungen mit langkettigen Kohlenstoffatomen, kann eine mehrstufige Destillation von Vorteil sein, bei der der gewünschte Strom für den Schritt c) als Kopfstrom der zweiten bzw. letzten Destillationskolonne abgenommen wird.

Die aus der Destillation in Schritt b) erhaltene Phase wird dann in Schritt c) einer Umsetzung in einer Reaktionseinheit zugeführt. Die Reaktionseinheit für die Umsetzung in Schritt c) kann aus einem oder mehreren Reaktoren bestehen. Der oder die Reaktor(en) können insbesondere aus der Gruppe, bestehend aus einem Rührkesselreaktor, einem Schlaufenreaktor, einem Jet-Loop-Reaktor, einem Blasensäulenreaktor oder Kombinationen daraus, ausgewählt sein. Sind mehrere Reaktoren vorhanden können die Reaktoren gleich oder unterschiedlich sein. Sind mehrere Reaktoren vorhanden, können diese parallel oder in Reihe geschaltet oder einer Mischform aus paralleler und serieller Schaltung angeordnet sein.

Eine im Rahmen der vorliegenden Erfindung besonders bevorzugte Ausführungsform ist ein Verfahren zur Umsetzung von Tributen, welches die folgenden Schritte umfasst:
a) Bereitstellen eines Tributen-Feedstroms, der zumindest lineare und verzweigte Isomere des Tributens umfasst;
b) Destillation des bereitgestellten Feedstroms in mindestens einer Destillationskolonne unter Erhalt zumindest einer Leichtsiederphase und einer Schwersiederphase, wobei 1 Gew.-% bis weniger als 50 Gew.-% des destillierten Feedstroms als Leichtsiederphase anfällt und wobei der Anteil an linearen Isomeren des Tributens in der Leichtsiederphase geringer ist als der Anteil der linearen Isomere des Tributens in der Schwersiederphase;
c) Zuführen der Schwersiederphase aus Schritt b) zu einer Reaktionseinheit, die einen oder mehrere Reaktoren umfasst, und Durchführen einer Umsetzung in der Reaktionseinheit, wobei die Umsetzung entweder eine heterogen katalysierte Hydroformylierung in Anwesenheit von Synthesegas, eine homogen katalysierte Hydroformylierung in Anwesenheit von Synthesegas oder eine homogen katalysierte Alkoxycarbonylierung in Anwesenheit von Kohlenmonoxid und einem C1- bis C6- Alkohol ist.

Ist die Umsetzung eine homogen katalysierte Reaktion sind folgende Verfahrensbedingungen bevorzugt:
Die bei dem Verfahren eingesetzten Olefine werden mit Synthesegas in Gegenwart eines homogen gelösten Katalysatorsystems hydroformyliert. Das molare Verhältnis zwischen Synthesegas und dem Einsatzgemisch sollte zwischen 6:1 und 1:1, vorzugsweise zwischen 3:1 und 1:1, besonders bevorzug zwischen 2:1 und 1:1 liegen. Die Hydroformylierung kann optional in Anwesenheit eines dem Fachmann bekannten Lösemittels durchgeführt werden.

Das bei der Hydroformylierung einsetzbare homogene Katalysatorsystem kann Co oder Rh, vorzugsweise Rh, und vorzugsweise einem phosphorhaltigen Liganden umfassen. Phosphorhaltige Liganden sind bei Co praktisch nicht unbedingt notwendig. Entsprechende Katalysatorsysteme sind dem Fachmann geläufig. In einer besonders bevorzugten Ausführungsform umfasst oder besteht das homogene Katalysatorsystem aus Rh und einem phosphorhaltigen Liganden. Geeignete Liganden für die erfindungsgemäßen Katalysatorsysteme sind dem Fachmann bekannt (siehe z. B. die Lehrbücher "Rhodium Catalyzed Hydroformylation" (aus 2002) von P. W. N. M van Leeuwen oder "Hydrofomylation - Fundamentals, Proceses and Applications in Organic Synthesis" (aus 2016) von A. Börner und R. Franke).

Der phosphorhaltige Ligand für das erfindungsgemäße Katalysatorsystem ist vorzugsweise ein Phosphin (z. B. TPP (Triphenylphosphin), ein Monophosphit (z. B. Alkanox 240 (Tris(2,4-di-tert-butylphenyl)phosphit) oder ein Bisphosphit (z. B. Biphephos). Es können auch Mischungen von Liganden eingesetzt werden.

Die Temperatur bei der homogen katalysierten Hydroformylierung liegt vorzugsweise im Bereich von 80 bis 250 °C, weiterhin bevorzugt im Bereich von 90 bis 225 °C und besonders bevorzugt im Bereich von 100 bis 210 °C. Der Druck bei der homogen katalysierten Hydroformylierung liegt vorzugsweise im Bereich von 20 bis 350 bar, weiterhin bevorzugt im Bereich von 30 bis 325 bar und besonders bevorzugt im Bereich von 45 bis 300 bar.

Der Druck bei der Hydroformylierung entspricht üblicherweise dem Gesamtgasdruck. Der Gesamtgasdruck meint im Rahmen der vorliegenden Erfindung die Summe der vorliegenden Drücke aller vorhandenen gasförmigen Stoffe, also den Druck der (gesamten) Gasphase. Im vorliegenden Verfahren entspricht dies insbesondere der Summe der Partialdrücke von CO und H₂, d. h. der Gesamtgasdruck ist dann der Synthesegasdruck.

Homogene katalysierte Hydroformylierungen können als Flüssigaustragsverfahren ("liquid recycle") oder als Gasaustragsverfahren ("gas recycle) betrieben werden. Beide Verfahrensvarianten sind dem Fachmann bekannt und in vielen Lehrbüchern beschrieben. Eine konkrete Auswahl eines solchen Verfahrens ist im Rahmen der vorliegenden Erfindung nicht notwendig, weil das Verfahren grundsätzlich auf beide Arten durchgeführt werden kann. Wichtig bei der homogenen Katalyse ist allenfalls noch die Abtrennung des Katalysatorsystems aus dem Reaktionsaustrag. Bei einem flüssigen Austrag ist dies beispielsweise über Flashverfahren oder Membrantrennung möglich. Bei einem gasförmigen Austrag beispielsweise mittels Kondensation und/oder Auswaschen. Auch dies ist dem Fachmann bekannt und darf keiner ausführlichen Erläuterung. Die weitere Aufarbeitung des Reaktionsaustrags, insbesondere die Abtrennung des Reaktionsprodukts, ist dem Fachmann ebenfalls geläufig und kann beispielsweise mittels eines thermischen Trennverfahrens wie der Destillation erfolgen. Thermische Trennung bzw. thermische Trennverfahren im Sinne der vorliegenden Erfindung meint ein Trennverfahren, bei dem die Trennung anhand des Siedepunktes erfolgt.

Ist die Umsetzung eine heterogen katalysierte Hydroformylierung sind folgende Verfahrensbedingungen bevorzugt:
Heterogen katalysierte Hydroformylierungen sind im Rahmen der vorliegenden Erfindung insbesondere solche, bei denen das Katalysatorsystem heterogenisiert wird, insbesondere durch Immobilisierung auf einem Trägermaterial (vgl. einleitende Diskussion in der WO 2015/028284 A1). Die Begriffe Heterogenisierung und Immobilisierung sind demnach so zu verstehen, dass das Katalysatorsystem durch Ausbildung eines dünnen Flüssigkeitsfilms auf der Oberfläche und/oder in den Poren eines festen Trägermaterials immobilisiert vorliegt.

Die heterogen katalysierte Hydroformylierung zeichnet sich insbesondere dadurch aus, dass die Schwersiederphase aus Schritt b) gasförmig über einen Support aus einem porösen keramischen Material, auf dem das Katalysatorsystem, welches ein Metall aus der 8. oder 9. Gruppe des Periodensystems der Elemente, mindestens einen organischen Phosphor-enthaltenden Liganden, einen Stabilisator umfasst, heterogenisiert vorliegt, geleitet wird.

Die Temperatur bei der heterogen katalysierten Hydroformylierung kann im Bereich von 65 bis 200 °C, vorzugsweise 75 bis 175 °C und besonders bevorzugt 85 bis 150 °C liegen. Der Druck bei der heterogen katalysierten Hydroformylierung sollte größer als 0 sein, aber insbesondere nicht größer als 35 bar, vorzugsweise nicht größer als 30 bar, besonders bevorzugt nicht größer 25 bar sein. Das molare Verhältnis zwischen Synthesegas und dem Einsatzgemisch sollte zwischen 6:1 und 1:1, vorzugsweise zwischen 5:1 und 3:1 liegen. Optional kann das Einsatzgemisch mit Inertgas oder einem Lösemittel verdünnt werden, beispielsweise mit den in technischen Kohlenwasserstoffströmen befindlichen Alkanen, um die Reaktion zu kontrollieren.

Das bei dem erfindungsgemäßen Hydroformylierungsverfahren eingesetzte Katalysatorsystem umfasst vorzugsweise ein Übergangsmetall aus der 8. oder 9. Gruppe des Periodensystems der Elemente, insbesondere Eisen, Ruthenium, Iridium, Cobalt oder Rhodium, besonders bevorzugt Cobalt und Rhodium, mindestens einen organischen Phosphor-enthaltenden Liganden, einen Stabilisator und optional eine ionische Flüssigkeit.

Der Stabilisator ist vorzugsweise eine organische Aminverbindung, besonders bevorzugt eine organische Aminverbindung, die mindestens eine 2,2,6,6-Tetramethylpiperidineinheit enthält.

Der organische Phosphor-enthaltende Ligand für das erfindungsgemäße Katalysatorsystem weist vorzugsweise die allgemeine Formel (I)

R' - A - R" - A - R‴ (I)

wobei R', R" und R‴ jeweils organische Reste sind und beide A jeweils eine brückende -O-P(-O)2-Gruppe sind, wobei zwei der drei Sauerstoffatome -O- jeweils an Rest R' und den Rest R‴ gebunden sind, mit der Maßgabe das R' und R‴ zwei voneinander getrennte organische Reste sind. R' und R‴ können den gleichen oder einen unterschiedlichen organischen Rest darstellen. Die organischen Reste R', R" und R‴ enthalten vorzugsweise keine endständige Trialkoxysilangruppe.

In einer bevorzugten Ausführungsform sind R', R" und R‴ in der Verbindung der Formel (I), vorzugsweise ausgewählt aus substituierten oder unsubstituierten 1,1'-Biphenyl-, 1,1'-Binaphthyl- und ortho-Phenylgruppen, insbesondere aus substituierten oder unsubstituierten 1,1'-Biphenylgruppen, mit der Maßgabe das R' und R‴ nicht identisch sind. Besonders bevorzugt weisen die substituierten 1,1'-Biphenylgruppen in 3,3'- und/oder 5,5'-Stellung des 1,1'-Biphenylgrundkörpers eine Alkylgruppe und/oder eine Alkoxygruppe auf, insbesondere eine C1-C4-Alkylgruppe, besonders bevorzugt eine tert.-Butyl- und/oder Methylgruppe und/oder bevorzugt einen C1-C5-Alkoxgruppe, besonders bevorzugt eine Methoxygruppe. Ein Beispiel für einen geeigneten Liganden ist Biphephos (6,6'-[(3,3'-Di-tert-butyl-5,5'-dimethoxy-1,1'-biphenyl-2,2'-diyl)bis(oxy)]bis(dibenzo[d,fJ[1,3,2]dioxaphosphepin)).

Das poröse keramische Material, aus dem der Support besteht, ist vorzugsweise ausgewählt aus der Gruppe, bestehend aus einer Silikatkeramik, einer oxidischen Keramik, einer nitridischen Keramik, einer carbidischen Keramik, einer silicidischen Keramik und Mischungen davon. Die Silikatkeramik ist vorzugsweise ausgewählt aus Alumosilikat, Magnesiumsilikat, und Mischungen davon, wie z. B. Betonit. Die oxidische Keramik ist vorzugsweise ausgewählt aus γ-Aluminiumoxid, α-Aluminiumoxid, Titandioxid, Beryliumoxid, Zirkoniumoxid, Aluminiumtitanat, Bariumtitanat, Zinkoxid, Eisenoxide (Ferrite) und Mischungen davon. Die nitridische Keramik ist vorzugsweise ausgewählt aus Siliciumnitrid, Bornitrid, Aluminiumnitrid und Mischungen davon. Die carbidische Keramik ist vorzugsweise ausgewählt aus Siliciumcarbid, Borcarbid, Wolframcarbid oder Mischungen davon. Denkbar sind auch Mischungen aus carbidischen und nitridischen Keramik, die sogenannten Carbonitride. Die silicidische Keramik ist vorzugsweise Molybdändisilicid. Der Support gemäß der vorliegenden Erfindung, auf den das Katalysatorsystem aufgebracht wird, besteht vorzugsweise aus einer carbidischen Keramik.

Der Support kann ein Monolith sein, d. h. der Support kann aus einem Block (ein dreidimensionales Objekt) aus einem keramischen Material bestehen. Ein solcher Block kann sowohl einstückig ausgebildet sein als auch aus mehreren, also mindestens zwei Einzelteilen bestehen, die zu dem Block zusammengefügt werden können und/oder miteinander fest oder lösbar verbunden sind. Der Support kann aber auch als in Form eines Granulats oder in Form von Pellets vorliegen. Der mittlere Partikeldurchmesser (d50) des Supports kann dann von 0,1 mm bis 7 mm, vorzugsweise 0,3 bis 6 mm, besonders bevorzugt von 0,5 mm bis 5 mm betragen. Der mittlere Partikeldurchmesser kann mittels bildgebender Verfahren ermittelt werden, insbesondere durch die in den Normen ISO 13322-1 (Stand: 2004-12-01) und ISO 13322-2 (Stand: 2006-11-01) genannten Verfahren ermittelt werden. Die Herstellung des Supports, in Form eines Granulats oder in Form von Pellets kann nach dem Fachmann bekannten Verfahren erfolgen. Beispielsweise könnte es dadurch erfolgen, dass ein Monolith aus dem carbidischen, nitridischen, silicidischen Material oder Mischungen davon mechanisch zerkleinert wird, beispielsweise mit einem Backenbrecher, und die Partikelgröße des erhaltenen Bruchgranulats mittels Sieben eingestellt wird.

Der Support besteht erfindungsgemäß aus einem porösen keramischen Material, das keramische Material weist also Poren auf. Grundsätzlich sind verschiedene Porositäten oder Porendurchmesser denkbar. Der Porendurchmesser liegt jedoch vorzugsweise im Bereich von 0,9 nm bis 30 µm, weiterhin bevorzugt im Bereich von 10 nm bis 25 µm und besonders bevorzugt im Bereich von 70 nm bis 20 µm. Der Porendurchmesser kann mittels Stickstoffadsorption oder Quecksilber-Porosimetrie gemäß DIN 66133 (Stand: 1993-06) bestimmt werden.

Auf den Support aus dem keramischen Material kann zusätzlich ein sogenannter Washcoat aufgetragen werden, welcher bezogen auf das keramische Material des Supports aus dem gleichen oder einem unterschiedlichen Keramikmaterial besteht, insbesondere ein aus den vorgenannten Keramikmaterialen ausgewähltes Keramikmaterial, vorzugsweise Siliciumoxid. Der Washcoat selber kann porös oder unporös sein, vorzugsweise ist der Waschcoat unporös. Die Partikelgröße des Washcoats beträgt vorzugsweise 5 nm bis 3 µm, vorzugsweise 7 nm bis 700nm. Der Washcoat wird verwendet, um die gewünschte Porengröße einzubringen oder zu generieren und/oder, um die Oberfläche des Supports zu erhöhen. Der Auftrag des Washcoats kann insbesondere mittels Eintauchen (Dipcoating) in eine Washcoat-Lösung, die das Keramikmaterial des Washcoats, ggf. auch als Precursor, enthält, erfolgen. Die Menge das auf dem Support befindlichen Waschcoats beträgt ≤ 20 Gew.-%, vorzugsweise ≤ 15 Gew.-%, besonders bevorzugt ≤ 10 Gew.-% bezogen auf die Gesamtmenge des Supports. Auf den so erzeugten keramischen Support mit dem aufgebrachten Washcoat wird dann das Katalysatorsystem aufgebracht. Es ist jedoch bevorzugt, dass der Support keinen Washcoat umfasst.

Der bei der heterogen katalysierten Hydroformylierung erhaltene Reaktionsaustrag muss grundsätzlich nicht vom Katalysatorsystem befreit werden. Die weitere Aufarbeitung, also beispielsweise die Abtrennung der Reaktionsprodukte, ist dem Fachmann geläufig und kann grundsätzlich destillativ oder nach einem anderen thermischen Trennverfahren erfolgen.

Ist die Umsetzung eine homogen katalysierte Alkoxycarbonylierung sind folgende Verfahrensbedingungen bevorzugt:
Bei der Alkoxycarbonylierung werden die Olefine des Feedstroms zusammen mit Kohlenmonoxid (CO) und einem Alkohol, im vorliegenden Fall einem C1- bis C6-Alkohol, in Anwesenheit eines homogen katalysierten Katalysatorsystems zur Reaktion gebracht und es entstehen Ester.

Das Kohlenmonoxid kann dabei direkt als Einsatzgemisch oder durch die Zugabe eines Kohlenmonoxidhaltigen Gases, ausgewählt aus Synthesegas, Wassergas, Generatorgas und anderen Kohlenmonoxidhaltigen Gasen bereitgestellt werden. Möglich ist es auch, das Kohlenmonoxid dadurch bereitzustellen, dass das Kohlenmonoxid-haltige Gas vorher in einer für den Fachmann bekannten Weise in seine Komponenten aufgetrennt wird und das Kohlenmonoxid zur Reaktionszone geleitet wird. Das Kohlenmonoxid kann dabei weiterhin einen gewissen Anteil an Wasserstoff oder anderen Gasen enthalten, weil eine vollständige Auftrennung kaum zu realisieren ist.

Der bei Alkoxycarbonylierung eingesetzte Alkohol ist ein Mono- oder Polyalkohol (Polyalkohol = zwei oder mehr OH-Gruppen) mit 1 bis 6 Kohlenstoffatomen. Geeignete Alkohole für die Umsetzung in Schritt c) sind Methanol, Ethanol, 1-Propanol, 1-Butanol, 1-Pentanol, 1-Hexanol, 2-Propanol, tert-Butanol, 3-Pentanol, Cyclohexanol, Phenol oder Mischungen daraus, Bevorzugt werden Methanol und Ethanol bei der Alkoxycarbonylierung eingesetzt. Wird Methanol eingesetzt, wird die Umsetzung auch als Methoxycarbonylierung bezeichnet. Bei Einsatz von Ethanol wird die Umsetzung auch als Ethoxycarbonylierung bezeichnet.

Das für die Alkoxycarbonylierung eingesetzte homogene Katalysatorsystem umfasst vorzugsweise zumindest ein Metall aus der Gruppe 8 bis 10 des Periodensystems der Elemente (PSE) oder eine Verbindung davon, einen phosphorhaltigen Liganden und eine Säure als Co-Katalysator.

Das Metall aus der Gruppe 8 bis 10 des PSE ist vorzugsweise Palladium. Das Palladium wird bevorzugt in Form einer Vorläuferverbindung als Palladiumverbindung eingesetzt, die durch den phosphorhaltigen Liganden koordiniert wird. Beispiele für Palladiumverbindungen, die als Vorläuferverbindungen eingesetzt werden können, sind Palladiumchlorid [PdCl₂], Palladium(II)-Acetylacetonat [Pd(acac)₂], Palladium(II)-Acetat [Pd(OAc)₂], Dichloro-(1,5-cyclooctadien)palladium(II) [Pd(cod)₂Cl₂], Bis(dibenzylideneaceton)palladium(0) [Pd(dba)₂], Tris(dibenzylideneaceton)dipalladium(0) [Pd₂(dba)₃] Bis(acetonitril)-dichloropalladium(II) [Pd(CH₃CN)₂Cl₂], Palladium(cinnamyl)-dichlorid [Pd(cinnamyl)Cl₂]. Vorzugsweise kommen die Verbindungen [Pd(acac)₂] oder [Pd(OAc)₂] zum Einsatz. Die Metallkonzentration von Palladium bei der Alkoxycarbonylierung beträgt vorzugsweise zwischen 0,01 und 0,6 Mol-%, bevorzugt zwischen 0,03 und 0,3 Mol-%, besonders bevorzugt zwischen 0,04 und 0,2 Mol-% bezogen auf die Stoffmenge des eingesetzten Kohlenwasserstoffs.

Geeignete phosphorhaltige Liganden des erfindungsgemäßen Katalysatorsystems weisen vorzugsweise eine Bidentat-Struktur auf. Bevorzugte phosphorhaltige Liganden für das erfindungsgemäße Katalysatorsystem sind benzolbasierte Diphosphinverbindungen, wie sie beispielsweise in der EP 3 121 184 A2 offenbart worden sind. Die Liganden können in einer Vorreaktion mit dem Palladium kombiniert werden, sodass der Palladium-Ligand-Komplex zur Reaktionszone geführt wird, oder in situ zur Reaktion gegeben und dort mit dem Palladium kombiniert werden. Das Molverhältnis von Ligand : Metall kann bei der Alkoxycarbonylierung 1 : 1 bis 10 : 1, vorzugsweise 2 : 1 bis 6 : 1, besonders bevorzugt 3 : 1 bis 5 : 1 betragen.

Das homogene Katalysatorsystem umfasst bei der Alkoxycarbonylierung weiterhin eine Säure, wobei es sich insbesondere um eine Brönsted- oder eine Lewis-Säure handeln kann. Als Lewis-Säure können insbesondere Aluminiumtriflat, Aluminiumchlorid, Aluminiumhydrid, Trimethylaluminium, Tris(pentafluorophenyl)boran, Bortrifluorid, Bortrichlorid oder Mischungen daraus eingesetzt werden. Von den genannten Lewis-Säuren wird bevorzugt Aluminiumtriflat eingesetzt. Die Lewis-Säure wird vorzugsweise in einem Molverhältnis Lewis-Säure : Ligand von 1 : 1 bis 20 : 1, vorzugsweise 2 : 1 bis 15 : 1, besonders bevorzugt 5 : 1 bis 10 : 1 hinzugegeben.

Geeignete Brönsted-Säuren haben vorzugsweise eine Säurestärke von pKs ≤ 5, besonders bevorzugt eine Säurestärke von pKs ≤ 3. Die angegebene Säurestärke pKs bezieht sich auf den bei Normalbedingungen (25°C, 1,01325 bar) bestimmten pKs-Wert. Bei einer mehrprotonigen Säure bezieht sich die Säurestärke pKs im Rahmen dieser Erfindung auf den pKs-Wert des ersten Protolyseschrittes. Die Brönsted-Säure wird vorzugsweise in einem Molverhältnis Brönsted-Säure : Ligand von 1 : 1 bis 15 : 1, vorzugsweise 2 : 1 bis 10 : 1, besonders bevorzugt 3 : 1 bis 5 : 1 hinzugegeben.

Als Brönsted-Säuren können insbesondere Perchlorsäure, Schwefelsäure, Phosphorsäure, Methylphosphonsäure oder Sulfonsäuren eingesetzt werden. Geeignete Sulfonsäuren sind beispielsweise Methansulfonsäure, Trifluormethansulfonsäure, tert-Butansulfonsäure, p-Toluolsulfonsäure (PTSA), 2-Hydroxypropan-2-sulfonsäure, 2,4,6-Trimethylbenzolsulfonsäure und Dodecylsulfonsäure. Besonders bevorzugte Säuren sind Schwefelsäure, Methansulfonsäure, Trifluormethansulfonsäure und p-Toluolsulfonsäure. Vorzugsweise handelt es sich bei der Säure um Schwefelsäure. Carbonsäure sind hingegen weniger bis gar nicht geeignet.

Die homogen katalysierte Alkoxycarbonylierung wird vorzugsweise bei einer Temperatur von 25 bis 140 °C, weiterhin bevorzugt bei einer Temperatur von 60 bis 120 °C und besonders bevorzugt bei einer Temperatur von 70 bis 110 °C durchgeführt. Der Druck kann zwischen 5 und 60 bar, vorzugsweise zwischen 10 und 40 bar, besonders bevorzugt zwischen 15 und 30 bar betragen.

Durch die homogen katalysierte Alkoxycarbonylierung wird ein Produktgemisch erhalten, die zumindest den durch die Umsetzung gebildeten Ester, das homogene Katalysatorsystem, Leichtsieder, beispielsweise leichtsiedende Nebenprodukte wie Ether, Hochsieder, nicht umgesetzte Alkohole und ggf. nicht umgesetzte Kohlenwasserstoffe umfasst. Das Produktgemisch kann deshalb einer nachfolgenden Katalysatorabtrennung unterworfen werden. Dies kann beispielsweise mit einer Membrantrennung erfolgen, wodurch das homogene Katalysatorsystem und nicht umgesetzter Kohlenwasserstoff und/oder nicht umgesetzter Alkohol im Retentat angereichert, während der gebildete Ester im Permeat angereichert wird. Das Retentat, welches das angereicherte homogene Katalysatorsystem umfasst, kann in die Reaktionszone zurückgeführt werden.

Die weitere Aufarbeitung des Permeats, insbesondere die Abtrennung der Ester als Zielprodukte, kann nach bekannten Verfahren erfolgen und ist dem Fachmann grundsätzlich geläufig. Eine Möglichkeit sind thermische Trennverfahren wie die Destillation.

Die bei der Destillation in Schritt b) anfallende Leichtsiederphase kann in einer bevorzugten Ausführungsform der vorliegenden Erfindung einer Hydrierung unterworfen werden. Dabei werden die in der Leichtsiederphase vorhandenen Isomere der eingesetzten Olefine zu den entsprechenden Alkanen hydriert. Die Hydrierung erfolgt in einer Hydriereinheit, die aus einem oder mehreren Reaktoren bestehen kann. Die Reaktoren können im geraden Durchgang oder in Kreislauffahrweise betrieben werden. In einer bevorzugten Ausführungsform der vorliegenden Erfindung umfasst die Hydriereinheit mindestens zwei Reaktoren. Vorzugsweise wird dabei der erste Reaktor im Kreislauf betrieben und der zweite Reaktor im geraden Durchgang. Der erste und der zweite Reaktor können hierbei über einen Überlauf miteinander verbunden sein. Das hat den Vorteil, dass zwischen dem ersten und dem zweiten Reaktor keine Pumpe eingesetzt werden muss. Bei der Hydrierung wird Wasserstoff vorzugsweise im stöchiometrischen Überschuss eingesetzt, besonders bevorzugt in einem stöchiometrischen Überschuss von 5 bis 30 %.

Die Hydrierung der Leichtsiederphase kann an geeigneten und bekannten Trägerkatalysatoren durchgeführt werden. Geeignete Trägerkatalysatoren umfassen zumindest ein Übergangsmetall aus der Gruppe bestehend aus Palladium, Platin, Rhodium. Ruthenium, Nickel oder Mischungen davon und ein Trägermaterial aus der Gruppe, bestehend aus Aluminiumoxid, Siliciumdioxid, Titandioxid, Magnesiumoxid oder Mischungen davon. In einer bevorzugten Ausführungsform der vorliegenden Erfindung wird bei der Hydrierung im optionalen Schritt ein Trägerkatalysator eingesetzt, der als Übergangsmetall Palladium oder Nickel enthält.

Die Hydrierung wird vorzugsweise bei einer Temperatur von 100 bis 180 °C, besonders bevorzugt bei einer Temperatur von 135 bis 160 °C durchgeführt. Der Druck beträgt bei der Hydrierung vorzugsweise 5 bis 40 barü, besonders bevorzugt 10 bis 30 barü. Der Druck wird hierbei insbesondere durch die Gasphase, also den Wasserstoff erzeugt. Die Hydrierung wird vorzugsweise in der Flüssigphase durchgeführt. Nach der Hydrierung kann eine dem Fachmann bekannte Phasentrennung erfolgen, um die Gasphase, die nicht umgesetzten Wasserstoff und ggf. auch geringe Mengen Kohlenwasserstoffe umfasst, von der flüssigen Phase abzutrennen.

### Beispiele

Hydroformylierung von Tributen
49 kg Tributen (Feed) wurden in einer Destillationskolonne (80 I) mit mehreren Packungsbetten (Montz A3-1000) bei einer Temperatur von 70 °C (Kopf) bzw. 110 °C (Sumpf) und einem Druck von 90 mbar (Kopf) bzw. 120 mbar (Sumpf) destilliert. Von den eingesetzten Tributen wurden 20 bis 25% als Destillat (= Leichtsieder) abgetrennt. Die restlichen 75 bis 80% verbleiben im Sumpf und sind daher Schwersieder.

Die drei unterschiedlichen Tributenströme Feed, Destillat und Sumpf wurden auf ihre Zusammensetzung untersucht. Da die Identifizierung der einzelnen Isomere von Tributen aufgrund der hohen Anzahl an Isomeren schwierig ist, wurden die Tributenströme auf den Anteil verschiedener Fraktionen mittels Gaschromatographie (GC-Kapillarsäule, Petrocol, DH 150) untersucht. Zur Vereinfachung wurden deshalb anhand ihrer Retentionszeiten identifiziert (s. auch Abbildung 1):
- Hochverzweigte Isomere mit Retentionzeiten von 70 bis 109 min,
- Mittelverzweigte Isomere mit Retentionszeiten von 109 bis 134 min, und
- Wenigverzweigte Isomere mit Retentionszeiten von 134 bis 180 min

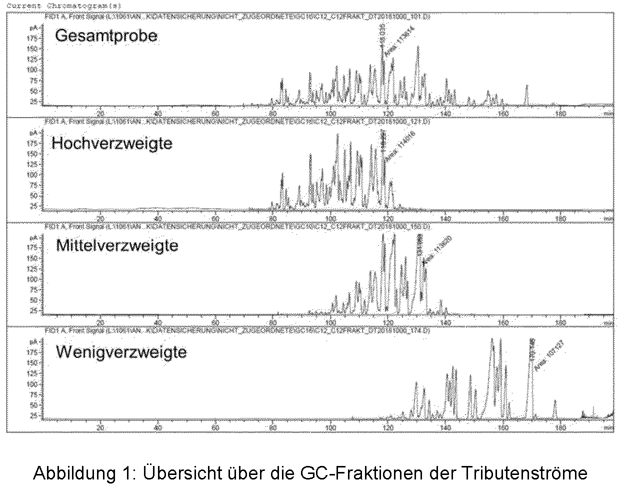

Eine Übersicht über den Anteil der jeweiligen o. g. Fraktionen in den Tributenströme ist in Tabelle 1 zu sehen.

**Tabelle 1: Anteil der Fraktionen in den jeweiligen Tributenströmen (Massen %)**

| | **Feed (vor Destillation)** | **Destillat (Leichtsieder)** | **Sumpf (Schwersieder)** |
|---|---|---|---|
| Hochverzweigte Isomere | 23,5 % | 93,5% | 0 % |
| Mittelverzweigte Isomere | 61 % | 6,5 % | 79 % |
| Wenigverzweigte Isomere | 15,5 % | 0% | 21 % |

Aus der Analyse von Feed, Destillat und Sumpf zeigt sich, dass durch die Destillation vor allem hochverzweigte Isomere des Tributens aus dem Feed abgetrennt werden und im Destillat landen. Der Anteil an wenigverzweigten Isomeren ist im Sumpf deutlich höher als im Feed oder im Destillat.

Mit den drei Tributenströmen Feed, Destillat und Sumpf wurden in 100 ml Autoklaven jeweils eine Hydroformylierung durchgeführt. Dabei wurde Rhodium (40 ppm Rh) mit einem Liganden (Alkanox 240) im 5-fachem molaren Überschuss ((Verhältnis Gesamtphosphor zu Rhodium) als Katalysator eingesetzt. Die Temperatur betrug 130 bis 150 °C. Die Hydroformylierung wurden weiterhin jeweils bei 235 bis 250 bar Synthesegasdruck (CO/H₂-Verhältnis = 1:1 (Vol.-%)) durchgeführt. Als Lösemittel wurden jeweils 40 bis 46 g Toluol verwendet. 10 Minuten, 60 Minuten und 180 Minuten nach Reaktionsstart wurden jeweils Proben genommen und auf den Reaktionsumsatz untersucht (Umsatz= Stoffmenge zum Zeitpunkt t/ Stoffmenge (Beginn der Reaktion)). Die Ergebnisse der Hydroformylierung sind in Tabelle 2 gezeigt.

**Tabelle 2: Reaktionsumsatz bei der Hydroformylierung**

| | **Umsatz / %** | | |
|---|---|---|---|
| | Nach 10 Minuten | Nach 60 Minuten | Nach 180 Minuten |
| Feed (vor Destillation) | 38,8 | 80,4 | 90,9 |
| Destillat (Leichtsieder) | 5,9 | 32.51 | 57,8 |
| Sumpf (Schwersieder) | 43,3 | 85.12 | 94,2 |

Aus der Tabelle 2 ist zu entnehmen, dass eine deutliche Beschleunigung der Reaktion durch eine vorherige Destillation erreicht werden kann. Verglichen mit dem Feed ist der Umsatz bei Einsatz des Sumpfstroms jeweils um etwa 5% höher.

## Patentansprüche

1. Verfahren zur Umsetzung von C11- bis C20-Olefinen, vorzugsweise von C12-, C16- oder C20-Olefinen, besonders bevorzugt von Tributen, welches die folgenden Schritte umfasst:
a) Bereitstellen eines C11- bis C20-Olefin-Feedstroms, vorzugsweise eines C12-, C16- oder C20-Olefin-Feedstroms, besonders bevorzugt eines Tributen-Feedstroms, der zumindest lineare und verzweigte Isomere der eingesetzten Olefine umfasst;
b) Destillation des bereitgestellten Feedstroms in mindestens einer Destillationskolonne unter Erhalt zumindest einer Leichtsiederphase und einer Schwersiederphase, wobei 1 Gew.-% bis weniger als 50 Gew.-% des destillierten Feedstroms als Leichtsiederphase anfällt und wobei der Anteil an linearen Isomeren in der Leichtsiederphase geringer ist als der Anteil der linearen Isomere in der Schwersiederphase;
c) Zuführen der Schwersiederphase aus Schritt b) zu einer Reaktionseinheit, die einen oder mehrere Reaktoren umfasst, und Durchführen einer Umsetzung in der Reaktionseinheit, wobei die Umsetzung entweder eine heterogen katalysierte Hydroformylierung in Anwesenheit von Synthesegas, eine homogen katalysierte Hydroformylierung in Anwesenheit von Synthesegas oder eine homogen katalysierte Alkoxycarbonylierung in Anwesenheit von Kohlenmonoxid und einem C1- bis C6-Alkohol ist.

2. Verfahren nach Anspruch 1, wobei die Umsetzung eine homogen katalysierte Hydroformylierung oder eine homogen katalysierte Alkoxycarbonylierung in Anwesenheit von Kohlenmonoxid und Methanol oder Ethanol ist.

3. Verfahren nach Anspruch 2, wobei das homogene Katalysatorsystem bei der Hydroformylierung Co oder Rh und vorzugsweise einem phosphorhaltigen Liganden umfasst.

4. Verfahren nach Anspruch 2, wobei das homogene Katalysatorsystem bei der Alkoxycarbonylierung ein Metall aus der Gruppe 8 bis 10 des Periodensystems der Elemente (PSE) oder eine Verbindung davon, einen phosphorhaltigen Liganden und eine Säure als Co-Katalysator umfasst.

5. Verfahren nach einem der vorhergehenden Ansprüche, wobei die in Schritt b) anfallende Leichtsiederphase einer Hydrierung unterworfen wird.

6. Verfahren nach Anspruch 5, wobei die Hydrierung bei einer Temperatur von 100 bis 180 °C durchgeführt wird.

7. Verfahren nach Anspruch 5 oder 6, wobei bei der Hydrierung Wasserstoff im stöchiometrischen Überschuss, vorzugsweise in einem stöchiometrischen Überschuss von 5 bis 30 % eingesetzt wird.

8. Verfahren nach einem der Ansprüche 5 bis 7, wobei bei der Hydrierung ein Trägerkatalysator, umfassend zumindest ein Übergangsmetall aus der Gruppe bestehend aus Palladium, Platin, Rhodium. Ruthenium, Nickel oder Mischungen davon und ein Trägermaterial aus der Gruppe, bestehend aus Aluminiumoxid, Siliciumdioxid, Titandioxid, Magnesiumoxid oder Mischungen davon, eingesetzt wird.

9. Verfahren nach einem der vorhergehenden Ansprüche, wobei die Destillation in Schritt b) bei einer Kopftemperatur im Bereich von 40 bis 100 °C, bevorzugt im Bereich von 55 bis 80 °C durchgeführt wird.

10. Verfahren nach einem der vorhergehenden Ansprüche, wobei der Druck am Kopf bei der Destillation in Schritt b) 50 bis 250 mbar, bevorzugt 80 bis 150 mbar beträgt.

11. Verfahren nach einem der vorhergehenden Ansprüche, wobei die Temperatur im Sumpf bei der Destillation in Schritt b) 90 bis 150 °C, bevorzugt 110 bis 140 °C beträgt.

12. Verfahren nach einem der vorhergehenden Ansprüche, wobei der Druck im Sumpf bei der Destillation in Schritt b) 70 bis 300 mbar, bevorzugt 90 bis 180 mbar beträgt.

13. Verfahren nach einem der vorhergehenden Ansprüche, wobei die mindestens eine Destillationskolonne in Schritt b) 20 bis 100 Böden, bevorzugt 30 bis 80 Böden umfasst.

14. Verfahren nach einem der vorhergehenden Ansprüche, wobei in Schritt b) 5 Gew.-% bis 40 Gew.-% des destillierten Feedstroms als Leichtsiederphase anfällt.

15. Verfahren nach Anspruch 14, wobei in Schritt b) 10 Gew.-% bis 30 Gew.-% des destillierten Feedstroms als Leichtsiederphase anfällt.
